(19) Europäisches Patentamt
European Patent Office
Office européen des brevets

(11) Veröffentlichungsnummer : **0 131 903**
**B1**

(12) # EUROPÄISCHE PATENTSCHRIFT

(45) Veröffentlichungstag der Patentschrift : **28.09.88**

(51) Int. Cl.⁴ : **C 07 D263/44**

(21) Anmeldenummer : **84108131.8**

(22) Anmeldetag : **11.07.84**

(54) **Verfahren zur Herstellung von N-(3,5-Dichlorphenyl)-oxazolidin-2,4-dionen.**

(30) Priorität : **16.07.83 DE 3325734**

(43) Veröffentlichungstag der Anmeldung : **23.01.85 Patentblatt 85/04**

(45) Bekanntmachung des Hinweises auf die Patenterteilung : **28.09.88 Patentblatt 88/39**

(84) Benannte Vertragsstaaten : **AT BE CH DE FR GB IT LI NL SE**

(56) Entgegenhaltungen : **DE-A- 2 324 591**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Zanker, Fritz, Dr.**
**Denkstrasse 27**
**D-6520 Worms 1 (DE)**
Erfinder : **Ohlinger, Rainer, Dr.**
**Ziegelhaeusser Landstrasse 31**
**D-6900 Heidelberg (DE)**

**EP 0 131 903 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von N-(3,5-Dichlorphenyl)-oxazolidin-2,4-dionen, bei dem im ersten Syntheseschritt der N-(3,5-Dichlorphenyl)-carbamoyloxi-essigsäureester in Gegenwart von Zinn (II) salzen von Carbonsäuren hergestellt und zusätzliche Mengen an N-(3,5-Dichlorphenyl)-oxazolidin-2,4-dionen aus der alkoholischen Mutterlauge durch Fällung mit Wasser abgetrennt werden.

Es ist bekannt, die Herstellung von N-(3,5-Dichlorphenyl)-oxazolidin-2,4-dionen in zwei Syntheseschritten vorzunehmen, wobei im ersten Schritt 3,5-Dichlorphenylisocyanat (= 3,5-DCI) und Glykolsäureester zu N-(3,5-Dichlorphenyl)-carbamoyloxi-essigsäureester umgesetzt werden, die im zweiten Schritt in Alkoholen in Gegenwart von Basen zu N-(3,5-Dichlorphenyl)-oxazolidin-2,4-dionen cyclisiert werden (DE-A-30 14 119). Die in fester Form anfallenden N-(3,5-Dichlorphenyl)-oxazolidin-2,4-dione werden in üblicher Weise, z. B. durch Filtration, von der Mutterlauge abgetrennt.

Bei der bekannten Umsetzung von gleichen Molen 3,5-DCI und Glykolsäure-estern entsteht ein Gemisch aus 85 bis 90 % N-(3,5-Dichlorphenyl)-carbamoyloxi-essigsäureestern (I) und 10 bis 15 % N, N'-Bis-(3,5-dichlorphenyl)-allophansäureestern (II), bezogen auf eingesetztes 3,5-DCI. Sowohl die N-(3,5-Dichlorphenyl)-carbamoyloxi-essigsäureester als auch die N,N'-Bis-(3,5-dichlorphenyl)-allophansäureester cyclisieren in Gegenwart basischer Katalysatoren zu N-(3,5-Dichlorphenyl)-oxazolidin-2,4-dionen (III), im Falle obigen Gemisches in ca. 90 % iger Ausbeute, bezogen auf eingesetztes 3,5-DCI.

Die Herstellung von N-(3,5-Dichlorphenyl)-oxazolidin-2,4-dionen aus 3,5-DCI und Glykolsäureestern wird anhand der Umsetzung von 3,5-DCI mit Vinyl-milchsäure-i-butylester wie folgt formelhaft wiedergegeben :

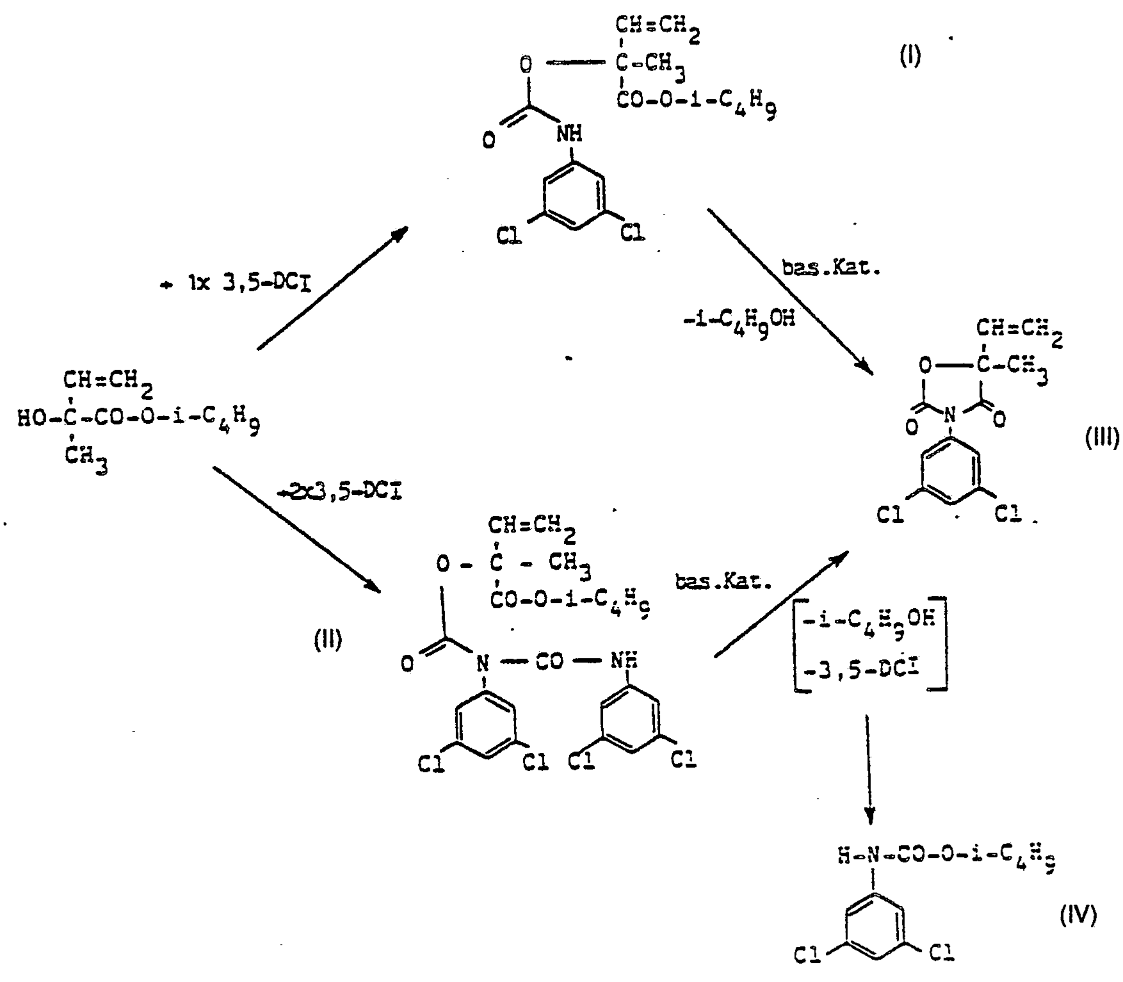

(I)
(II)
(III)
(IV)

Man erkennt, daß für die Bildung eines Mols N,N'-Bis-(3,5-dichlorphenyl)-allophansäureester (II) pro Mol Glykolsäureester 2 Mole 3,5-DCI notwendig sind, von denen bei der basenkatalysierten Cyclisierung nur ein Mol zu den gewünschten N-(3,5-Dichlorphenyl)-oxazolidin-2,4-dionen (III) weiterreagiert, während das andere Mol 3,5-DCI durch Reaktion mit den aus den Glykolsäureestern stammenden Alkoholen über das Allophansäurederivat II zu N-(3,5-Dichlorphenyl)-alkylcarbamaten IV umgesetzt wird und damit für die

Herstellung der Oxazolidine III verlorengeht. Bei der Verwendung der Ausgangsprodukte Glykolsäureester und 3,5-DCI im Molverhältnis 1 :1 geht bei der Entstehung des Allophansäurederivates II jedoch nicht nur 1 Mol 3,5-DCI, sondern auch 1 Mol Glykolsäureester, der an der chemischen Umsetzung nicht teilnimmt, für die Herstellung des Oxazolidin-2,4-dions verloren.

Die Ausbeute an N-(3,5-Dichlorphenyl)-oxazolidin-2,4-dionen wird somit durch die Bildung der N,N'-Bis-(3,5-dichlorphenyl)-allophansäureester stark herabgesetzt.

Aus DE-A-23 24 591 ist es bekannt, bei der Herstellung von N-(3,5-Dichlorphenyl)-carbamoyloxiessigsäureestern Dibutyl-zinn-diacetat, d.h. ein Salz des vierwertigen Zinns, in einer Menge von ca. 1,5 %, bezogen auf eingesetztes 3,5-DCI, als Katalysator zu verwenden. Die Ausbeute an N-(3,5-dichlorphenyl)-oxazolidin-2,4-dionen wird durch die Verwendung von Dibutyl-zinn-dilaurat anstelle von Dibutyl-zinn-diacetat als Katalysator nicht über 90 % erhöht. Gemäß DE-A-22 07 576, wo ohne Katalysator gearbeitet wird, erreicht man sogar eine höhere Ausbeute an N-(3,5-Dichlorphenyl)-oxazolidin-2,4-dionen als gemäß DE-A-23 24 591, wobei aber die Ausbeuten sowohl gemäß DE-A-22 07 576 als auch gemäß DE-A-23 24 591 kleiner als 90 % sind.

Die N-(3,5-Dichlorphenyl)-oxazolidin-2,4-dione fallen beim Arbeiten gemäß DE-A-30 14 119 in fester Form in alkoholischer Mutterlauge an, aus der sie z. B. durch Filtration abgetrennt werden. Die Mutterlaugen enthalten weitere Mengen an gelösten N-(3,5-Dichlorphenyl)-oxazolidin-2,4-dionen. Dadurch daß sie außerdem beträchtliche Mengen an N-(3,5-Dichlorphenyl)-alkylcarbamaten und nicht umgesetzten Glykolsäureestern enthalten, ist die Abtrennung der in der Mutterlauge gelösten N-(3,5-Dichlorphenyl)-oxazolidine sehr schwer. So bilden sich bei der Fällung der N-(3,5-Dichlorphenyl)-oxazolidin-2,4-dione aus der Mutterlauge mit Wasser N-(3,5-Dichlorphenyl)-oxazolidin-2,4-dion-haltige Schmieren oder schmierige Feststoffe, die sich nur sehr schwer abtrennen lassen und zu Filterverstopfungen führen, die nur schwer zu beseitigen sind. Die schmierigen Filterrückstände lassen sich nur unter beträchtlichen Schwierigkeiten und Produktverlusten zu N-(3,5-Dichlorphenyl)-oxazolidin-2,4-dionen technischer Reinheit aufarbeiten. Die Abtrennung von N-(3,5-Dichlorphenyl)-oxazolidin-2,4-dionen aus den Mutterlaugen ist daher technisch nicht sinnvoll.

Es wurde nun gefunden, daß man N-(3,5-Dichlorphenyl)-oxazolidin-2,4-dione der Formel

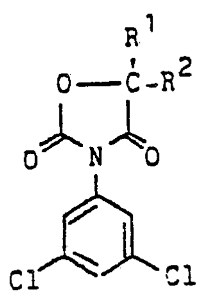

in der $R^1$ Halogenalkenyl mit 2 C-Atomen oder Alkenyl mit 2 C-Atomen, Chlormethyl, CN, CO-O-Alkyl mit 2 Kohlenstoffatomen, Alkoxialkyl oder Alkylthioalkyl mit je 2 bis 4 Kohlenstoffatomen und $R^2$ Halogenalkenyl mit 2 C-Atomen oder Alkenyl mit 2 C-Atomen, Wasserstoff oder Methyl bedeuten, in hoher Ausbeute erhält, wenn man 3,5-DCI mit Glycolsäureestern der Formel

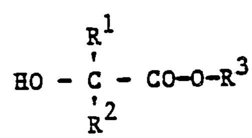

in der $R^1$ und $R^2$ die obige Bedeutung haben und $R^3$ einen Alkylrest mit 1 bis 10 Kohlenstoffatomen oder einen Cyclohexylrest bedeutet, in Gegenwart von Zinn (II) salzen der Formel

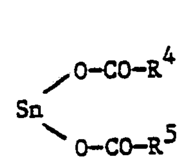

in der $R^4$ und $R^5$ gleich oder verschieden sind und Wasserstoff, einen Alkylrest mit 1 bis 17 Kohlenstoffatomen, einen Alkenylrest mit 2 bis 17 Kohlenstoffatomen, einen gegebenenfalls alkylierten Cycloalkylrest mit 5 bis 12 Kohlenstoffatomen, Benzyl, einen Phenylrest oder $R^4$ und $R^5$ zusammen $-CH_2-CH_2-$, $-CH=CH-$ oder o-Phenylen bedeuten, bei Temperaturen zwischen 0 und 110 °C umsetzt, in Gegenwart von Alkoholen der Formel

$R^6$—OH

in der $R^6$ einen Alkylrest mit 1 bis 10 Kohlenstoffatomen, Hydroxi-ethyl oder einen Cyclohexylrest bedeutet und gleich oder verschieden von $R^3$ ist, und basischen Katalysatoren erwärmt und bei Temperaturen zwischen —30 und +90 °C die aus der Reaktionsmischung ausgefallenen N-(3,5-Dichlorphenyl)-oxazolidin-2,4-dione von der Mutterlauge abtrennt und aus der Mutterlauge durch Zusatz von Wasser zusätzliche Mengen an N-(3,5-Dichlorphenyl)-oxazolidin-2,4-dionen ausfällt und diese bei Temperaturen zwischen —10 und +60 °C von der wäßrigen Flüssigkeit abtrennt.

Bevorzugte Substituenten $R^1$, $R^2$, $R^3$, $R^4$, $R^5$ und $R^6$ sind :

$R^1$ Vinyl und Methoximethyl, $R^2$ Methyl, $R^3$ und $R^6$ Alkyl mit 1 bis 4 Kohlenstoffatomen, wie Ethyl, n-Propyl-, i-Propyl, n-Butyl, sek-Butyl, tert.-Butyl, insbesondere Methyl und i-Butyl, $R^4$ und $R^5$ Alkylreste wie Methyl, Undecyl, Pentadecyl, Heptadecyl, insbesondere Heptyl, Alkenylreste wie Heptadecenyl, Cycloalkylreste wie Cyclohexyl.

Durch den Zusatz von Zinn (II) salzen nach dem Verfahren der Erfindung steigt der Umsatz zu den N-(3,5-Dichlorphenyl)-carbamoyloxi-essigsäureestern bei stöchiometrischem Einsatz von 3,5-DCI und Glykolsäureestern von 89 bis 91 % ohne Zinnsalze gemäß DE-A-30 14 119 bis auf ca. 96 %, bezogen auf eingesetztes 3,5-DCI, an ; die Entstehung der unerwünschten N,N'-Bis-(3,5-dichlorphenyl)-allophansäureester fällt dementsprechend von 10 bis 15 % auf ca. 2 %, bezogen auf eingesetztes 3,5-DCI, ab. Dadurch steigt die Ausbeute an N-(3,5-Dichlorphenyl)-oxazolidin-2,4-dionen ohne Aufarbeitung der Mutterlauge von ca. 90 bis auf ca. 96 %, und die Gesamtausbeute, d. h. mit Aufarbeitung der Mutterlauge, auf ca. 98 %, bezogen auf eingesetztes 3,5-DCI, an. Dabei arbeitet man drucklos (1 bar) oder unter Druck (1 bis 10 bar) bei Temperaturen zwischen 0 und 110 °C, vorzugsweise bei Temperaturen zwischen 10 und 90 °C.

Im Vergleich zu dem bekannten Verfahren (DE-A-30 14 119), das bei optimalen Temperaturen von ca. 120 °C abläuft, ist das Verfahren nach der Erfindung erheblich weniger energieaufwendig, da es bei niedrigerer Temperatur durchgeführt wird.

Die verwendete Menge an Zinn (II) salzen von Carbonsäuren beträgt 0,001 bis 1, insbesondere 0,005 bis 0,5 Gew.%, bezogen auf eingesetztes 3,5-DCI.

Man setzt 3,5-DCI und Glykolsäureester im Molverhältnis von 1,0 :0,95 bis 0,95 :1, vorzugsweise im Molverhältnis von 1 :1 ein. Da die Umsetzung selbst bei mäßig erhöhten Temperaturen rasch abläuft, eignet sich hierfür eine kontinuierliche Vermischung der Reaktionskomponenten in einer Mischdüse in Gegenwart katalytischer Mengen von Zinn (II) salzen von Carbonsäuren, die man vorteilhaft einer Reaktionskomponente beigemischt hat. Man kann die Umsetzung aber auch diskontinuierlich durchführen, z.B. durch Zulauf der Schmelze von 3,5-DCI zu mit katalytischen Mengen von Zinn (II) salzen von Carbonsäuren versetzten Glykolsäureestern in einem Rührgefäß.

Vorteilhaft führt man die durch Zinn (II) Salze von Carbonsäuren katalysierte Umsetzung von 3,5-DCI und Glykolsäureestern zu N-(3,5-Dichlorphenyl)-carbamoyloxi-essigsäureester lösungsmittelfrei durch.

Sie kann aber auch in Gegenwart von aprotischen organischen Lösungsmitteln, wie eventuell alkylierten oder chlorierten aromatischen Kohlenwasserstoffen, aliphatischen oder cycloaliphatischen Kohlenwasserstoffen, Estern wie Essigsäureethylester, Ethern wie Diethylether, cyclischen Ethern wie Tetrahydrofuran, Acetonitril oder Dimethylformamid oder in Gemischen dieser Lösungsmittel durchgeführt werden, was jedoch bei der Weiterverarbeitung zu N-(3,5-Dichlorphenyl)-oxazolidin-2,4-dionen keine Vorteile bringt, weil diese nach bekannten Verfahren vorteilhaft in Alkohol durchgeführt wird, so daß man gezwungen wäre, vor der Weiterverarbeitung der N-(3,5-Dichlorphenyl)-carbamoyloxiessigsäureester zu N-(3,5-Dichlorphenyl)-oxazolidin-2,4-dionen das aprotische Lösungsmittel kostenaufwendig zu entfernen.

Ein großer Vorteil des Verfahrens nach der Erfindung besteht darin, daß die alkoholischen Mutterlaugen der N-(3,5-Dichlorphenyl)-oxazolidin-2,4-dione nur geringe Anteile an Verunreinigungen enthalten, die die einfache Gewinnung der in den Mutterlaugen gelösten (N-3,5-Dichlorphenyl)-oxazolidin-2,4-dione durch Fällung mit Wasser und Filtration nicht stören. Dadurch erst wird die Aufarbeitung der Mutterlaugen technisch sinnvoll. Man verrührt diese bei Temperaturen zwischen —10 und +60 °C, insbesondere zwischen +10 und +40 °C mit Wasser, vorteilhaft im Volumenverhältnis von 1 : 10 bis 10 : 1, insbesondere im Volumenverhältnis von 1 :3 bis 3 :1 und saugt die ausgefallenen N-(3,5-Dichlorphenyl)-oxazolidin-2,4-dione ab. Ca. 2 % N-(3,5-Dichlorphenyl)-oxazolidin-2,4-dione lassen sich so zusätzlich gewinnen. Diese enthalten praktisch keine der oben erwähnten Verunreinigungen wie bei bekannten Verfahren anfallende Mutterlaugen und sind von einer Reinheit, die den technischen Anforderungen und den Anforderungen des Marktes genügt.

Die Benutzung von Wasser als Fällmittel für die N-(3,5-Dichlorphenyl)-oxazolidin-2,4-dione der alkoholischen Mutterlaugen und die gute Filtrierbarkeit der ausgefällten N-(3,5-Dichlorphenyl)-oxazolidin-2,4-dione erlauben es, deren Isolierung in den normalen Produktionsprozeß in Chargen von N-(3,5-Dichlorphenyl)-oxazolidin-2,4-dionen in folgender Weise zu integrieren : Die mit Wasser ausgefällten N-(3,5-Dichlorphenyl)-oxazolidin-2,4-dione aus der Mutterlauge der vorangegangenen Charge werden auf einer Nutsche abgesaugt, mit Alkoholen der Formel $R^6$—OH gewaschen und verbleiben auf der Nutsche. Die Waschalkohole werden verworfen. Hierzu gibt man die alkoholische Suspension der N-(3,5-Dichlorphenyl)-oxazolidin-2,4-dione in der Mutterlauge der laufenden Charge, saugt ab und wäscht mit geringen Mengen Alkoholen der Formel $R^6$—OH nach. Mutterlauge und Waschalkohole werden vereinigt

und später mit Wasser verdünnt. Der Nutscheninhalt, bestehend aus den vereinigten N-(3,5-Dichlorphenyl)-oxazolidin-2,4-dionen der laufenden Charge und denen der Aufarbeitung der Mutterlauge der vorangegangenen Charge, werden mit Wasser gewaschen und aus der Nutsche entfernt. Die Waschwässer werden verworfen.

Der Vorteil der Abtrennung der wertvollen N-(3,5-Dichlorphenyl)-oxazolidin-2,4-dione aus der Mutterlauge nach dem Verfahren der Erfindung unter Verzicht auf die bekannte Rückgewinnung der Alkohole $R^3$—OH und $R^6$—OH aus den Mutterlaugen ist beträchtlich, weil die Ausbeute an Endprodukt erhöht wird und demgegenüber der Verlust an Alkoholen vernachlässigt werden kann.

Die folgenden Beispiele erläutern die Durchführung des erfindungsgemäßen Verfahrens. Darin bedeuten die aufgeführten Teile Gewichtsteile. Sie verhalten sich zu den Volumenteilen wie Kilogramm zu Liter.

## Beispiel 1

Zu einer Mischung aus 146,2 Teilen Vinylmilchsäure-i-butylester und 0,1 Teilen Zinn-dilaurat wurden 160 Teile 40 °C warmes flüssiges 3,5-DCI (Schmp. 30 bis 31 °C) innerhalb von 10 Minuten so zugesetzt, daß dabei die Reaktionstemperatur von 35 auf 90 °C anstieg. Man kühlte auf 80 °C, rührte bei dieser Temperatur 2 Stunden nach und kühlte danach auf 70 °C. Man gab dann eine Lösung von 7 Teilen Tri-n-propylamin in 200 Volumenteilen Methalnol zu, hielt 5 Stunden bei 70 °C und kühlte auf Raumtemperatur ab. Das ausgefallene N-(3,5-Dichlorphenyl)-5-methyl-5-vinyl-oxazolidin-2,4-dion wurde abgesaugt, 2x mit je 30 Volumenteilen Methanol gewaschen und getrocknet. Ausbeute 229,5 Teile, entsprechend 94,3 % d. Th. bezogen auf eingesetztes 3,5-DCI. Schmp. 107 bis 108 °C.

Die Mutterlauge des abgesaugten N-(3,5-Dichlorphenyl)-5-methyl-5-vinyl-oxazolidin-2,4-dions wurde mit 200 Teilen Wasser versetzt, wobei die Temperatur unter 40 °C gehalten wurde. Es wurde abgesaugt, mit 15 Teilen Methanol gewaschen und getrocknet. Ausbeute 6,2 Teile N-(3,5-Dichlorphenyl)-5-methyl-5-vinyl-oxazolidin-2,4-dion, entsprechend 2,5 % d. Th., bezogen auf eingesetztes 3,5-DCI. Schmp. 105 bis 107 °C.

## Beispiel 2

Eine Mischung aus 146,2 Teilen Vinylmilchsäure-butylester und 0,1 Teilen Zinn-diacetat wurde mit 160 Teilen 3,5-DCI bei 60 °C versetzt. Beim Erwärmen auf 65 °C trat eine stark exotherme Reaktion ein, wodurch die Reaktionsmischung sich auf 90 °C erwärmte. Man hielt 3 Stunden bei dieser Temperatur, kühlte auf 70 °C ab, goß die Reaktionsmischung in eine Lösung von 6 Teilen Triethylamin in 200 Volumenteilen Methanol ein, hielt 5 Stunden bei 70 °C und kühlte danach auf 20 °C ab. Man saugte ab, wusch den Filterrückstand 2x mit je 30 Volumenteilen Methanol und trocknete ihn. Ausbeute 228,5 Teile N-(3,5-Dichlorphenyl)-5-methyl-5-vinyl-oxazolidin-2,4-dion, entsprechend 93,9 % d. Th., bezogen auf eingesetztes 3,5-DCI. Schmp. 106 bis 108 °C.

Die Mutterlauge (30 °C) wurde in 300 Teile Wasser gegeben. Es wurde abgesaugt, der Filterrückstand mit 12 Teilen Methanol gewaschen und getrocknet. Ausbeute 6 Teile N-(3,5-Dichlorphenyl)-5-methyl-5-vinyl-oxazolidin-2,4-dion, entsprechend 2,5 % d. Th., bezogen auf eingesetztes 3,5-DCI. Schmp. 104 bis 107 °C.

## Beispiel 3

Zu einer Lösung von 0,05 Teilen Zinn-dioctoat und 160,7 Teilen Methoximethylmilchsäure-i-butylester wurden bei 80 °C 160 Teile einer 40 °C warmen 3,5-DCI Schmelze innerhalb von 60 Minuten gegeben. Man rührte diese Reaktionsmischung weitere 2 Stunden bei 80 °C und ließ sie in eine Lösung von 5 Teilen Triethylamin in 180 Volumenteilen Methanol einfließen. Man hielt 5 Stunden bei 70 °C und kühlte auf 20 °C ab. Man saugte ab, wusch den Filterrückstand mit 2x 25 Teilen Methanol und trocknete ihn. Ausbeute 245 Teile N-(3,5-Dichlorphenyl)-5-methyl-5-methoximethyl-oxazolidin-2,4-dion, entsprechend 94,7 % d. Th., bezogen auf eingesetztes 3,5-DCI. Schmp. 111 bis 113 °C.

Die Mutterlauge des abgesaugten N-(3,5-Dichlorphenyl)-5-methyl-5-methoximethyl-oxazolidin-2,4-dions wurde bei 25 °C mit 350 Teilen Wasser versetzt. Es wurde abgesaugt, der Filterrückstand mit 15 Teilen Methanol gewaschen und getrocknet. Ausbeute 5,7 Teile N-(3,5-Dichlorphenyl)-5-methyl-5-methoximethyl-oxazolidin-2,4-dion, entsprechend 2,2 % d. Th., bezogen auf eingesetztes 3,5-DCI. Schmp. 108 bis 111 °C.

## Beispiel 4

Zu einer Lösung von 0,05 Teilen Zinn-dioctoat und 160 Teilen 3,5-DCI wurden 147 Teile Vinylmilchsäure-i-butylester innerhalb von 5 Minuten bei 60 °C zugesetzt. Man rührte 4 Stunden bei 60 °C nach und gab bei dieser Temperatur eine Lösung von 7 Teilen Tri-n-propylamin in 200 Volumenteilen Methanol zu. Man hielt 5 Stunden bei 70 °C, kühlte auf 20 °C ab, saugte das ausgefallene N-(3,5-Dichlorphenyl)-5-methyl-5-vinyl-oxazolidin-2,4-dion ab, wusch es mit 2x 20 Volumenteilen Methanol und trocknete es.

Ausbeute 234 Teile, entsprechend 96,1 % d. Th., bezogen auf eingesetztes 3,5-DCI. Schmp. 107 bis 108 °C.

Die Mutterlauge des abgesaugten N-(3,5-Dichlorphenyl)-5-methyl-5-vinyloxazolidin-2,4-dions wurde bei 25 °C mit 350 Teilen Wasser versetzt. Das ausgefallene N-(3,5-Dichlorphenyl)-oxazolidin-2,4-dion wurde abgesaugt, mit 2x 10 Teilen Methanol gewaschen und getrocknet. Ausbeute 5,6 Teile, entsprechend 2,3 % d. Th., bezogen auf eingesetztes 3,5-DCI. Schmp. 105 bis 107 °C.

Beispiel 5

Zu einer 20 °C warmen Lösung von 0,5 Teilen Zinn-dioctoat in 2 287 Teilen Vinylmilchsäure-i-butylester ließ man eine 50 °C warme Schmelze von 2 500 Teilen 3,5-DCI innerhalb von 5 Stunden so zulaufen, daß die Reaktionstemperatur von 20 auf 80 °C anstieg. Man hielt 2 Stunden bei 80 °C und ließ die Mischung in eine Lösung von 110 Teilen Tripropylamin in 3 200 Volumenteilen Methanol einlaufen, wobei die methanolische Reaktionslösung zum schwachen Sieden kam. Man erhitzte dann 4 Stunden am Rückfluß, kühlte auf unter 20 °C ab und saugte den ausgefallenen Niederschlag auf einer Nutsche ab. Man wusch ihn mit 300 Volumenteilen Methanol. Mutterlauge und Waschmethanol wurden vereinigt. Der Nutscheninhalt wurde weiterhin mit 10 000 Teilen Wasser gewaschen, gut abgesaugt und die Nutsche entleert. Ausbeute, bezogen auf Trockengewicht 3 600 Teile N-(3,5-Dichlorphenyl)-5-methyl-5-vinyl-oxazolidin-2,4-dion, entsprechend 94,6 % d. Th., bezogen auf eingesetztes 3,5-DCI. Die Waschwasser wurden verworfen.

Die mit dem Waschmethanol vereinigte Mutterlauge wurde unter Rühren mit 3 000 Teilen Wasser versetzt, wobei die Temperatur 35 °C nicht überstieg. Man saugte den entstandenen Niederschlag auf obiger ausgeräumter Nutsche ab und wusch ihn mit 50 Teilen Methanol. Die wäßrigen Filtrate und das Waschmethanol wurden verworfen. Der methanolfeuchte Niederschlag enthielt durchschnittlich 80 Teile an festem N-(3,5-Dichlorphenyl)-5-vinyl-oxazolidin-2,4-dion, entsprechend einer Ausbeute von 2,1 % d. Th., bezogen auf eingesetztes 3,5-DCI. Bei laufendem Chargenbetrieb wurde er nicht ausgetragen sondern auf der Nutsche mit der methanolischen Maische von N-(3,5-Dichlorphenyl)-5-methyl-5-vinyl-oxazolidin-2,4-dion der folgenden Charge verrührt und abgesaugt und der Filterrückstand, wie oben beschrieben mit 300 Volumenteilen Methanol und 10 000 Teilen Wasser gewaschen, abgesaugt und ausgetragen, wobei man wiederum eine mit dem Waschmethanol vereinigte Mutterlauge erhielt, die, wie oben beschrieben, aufgearbeitet wurde. Man erhielt so einen wasserfeuchten Nutschenaustrag, bestehend aus 3 600 + 80 = 3 680 Teilen N-(3,5-Dichlorphenyl)-5-methyl-5-vinyl-oxazolidin-2,4-dion, bezogen auf Trockengewicht, entsprechend einer 96,7 %igen Ausbeute, bezogen auf eingesetztes 3,5-DCI. Schmp. der Trockenware 106 bis 108 °C.

**Patentansprüche**

1. Verfahren zur Herstellung von N-(3,5-Dichlorphenyl)-oxazolidin-2,4-dionen der Formel

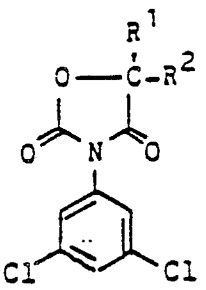

in der $R^1$ Halogenalkenyl mit 2 C-Atomen oder Alkenyl mit 2 C-Atomen, Chlormethyl, CN, CO-O-Alkyl mit 2 bis 5 Kohlenstoffatomen, Alkoxialkyl oder Alkylthioalkyl mit je 2 bis 4 Kohlenstoffatomen und $R^2$ Halogenalkenyl mit 2 C-Atomen, Alkenyl mit 2 C-Atomen, Wasserstoff oder Methyl bedeuten, dadurch gekennzeichnet, daß man 3,5-Dichlorphenylisocyanat mit Glykolsäureestern der Formel

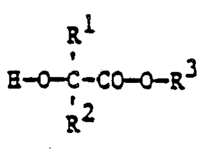

in der $R^1$ und $R^2$ die obige Bedeutung haben und $R^3$ einen Alkylrest mit 1 bis 10 Kohlenstoffatomen oder einen Cyclohexylrest bedeutet, in Gegenwart von Zinn(II)salzen der Formel

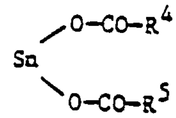

in der $R^4$ und $R^5$ gleich oder verschieden sind und Wasserstoff, einen Alkylrest mit 1 bis 17 Kohlenstoffatomen, einen Alkenylrest mit 2 bis 17 Kohlenstoffatomen, einen gegebenenfalls alkylierten Cycloalkylrest mit 5 bis 12 Kohlenstoffatomen, Benzyl oder einen Phenylrest bedeuten oder $R^4$ und $R^5$ zusammen $—CH_2—CH_2—$, —CH=CH— oder o-Phenylen bedeuten, bei Temperaturen zwischen 0 und 110 °C umsetzt, in Gegenwart von Alkoholen der Formel

$$R^6—OH$$

in der $R^6$ einen Alkylrest mit 1 bis 10 Kohlenstoffatomen, Hydroxiethyl, oder einen Cyclohexylrest bedeutet und gleich oder verschieden von $R^3$ ist, und basischen Verbindungen erwärmt, bei Temperaturen zwischen — 30 und + 90 °C die aus der Reaktionsmischung ausgefallenen N-(3,5-Dichlorphenyl)-oxazolidin-2,4-dione von der Mutterlauge abtrennt, und aus der alkoholischen Mutterlauge durch Zusatz von Wasser zusätzliche Mengen an N-(3,5-Dichlorphenyl)-oxazolidin-2,4-dionen ausfällt und diese bei Temperaturen zwischen — 10 und + 60 °C von der wäßrigen Flüssigkeit abtrennt.

2. Verfahren nach Anspruch 1, dadurch gekennzeichnet, daß man die Umsetzung mit Verbindungen durchführt, in denen $R^1$ Vinyl oder Methoximethyl, $R^2$ Methyl und $R^3$ Alkyl mit 1 bis 4 Kohlenstoffatomen bedeuten.

3. Verfahren nach Anspruch 1, dadurch gekennzeichnet, daß $R^4$ und $R^5$ Heptyl bedeuten.

4. Verfahren nach Anspruch 1, dadurch gekennzeichnet, daß das Volumenverhältnis von alkoholischer Mutterlauge zu Wasser 1 : 3 bis 3 : 1 ist.

5. Verfahren nach Anspruch 1, dadurch gekennzeichnet, daß man bei Chargenproduktion auf der Filtriereinrichtung die durch Wasserzusatz abgetrennten und abfiltrierten N-(3,5-Dichlorphenyl)-oxazolidin-2,4-dione der letzten Charge mit der Mischung aus Mutterlauge und N-(3,5-Dichlorphenyl)-oxazolidin-2,4-dionen der laufenden Charge mischt und gemeinsam durch Filtration von der Mutterlauge der laufenden Charge abtrennt.

**Claims**

1. A process for the preparation of an N-(3,5-dichlorophenyl)-oxazolidine-2,4-dione of the formula

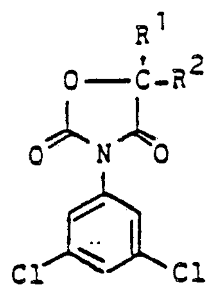

where $R^1$ is haloalkenyl or alkenyl, each of 2 carbon atoms, chloromethyl, CN, CO-O-alkyl of 2 to 5 carbon atoms, or alkoxyalkyl or alkylthioalkyl, each of 2 to 4 carbon atoms, and $R^2$ is haloalkenyl or alkenyl, each of 2 carbon atoms, hydrogen or methyl, wherein 3,5-dichlorophenyl isocyanate is reacted with a glycolate of the formula

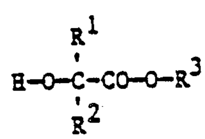

where $R^1$ and $R^2$ have the above meanings and $R^3$ is alkyl of 1 to 10 carbon atoms or cyclohexyl, in the presence of a tin(II) salt of the formula

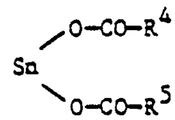

where $R^4$ und $R^5$ are identical or different and are each hydrogen, alkyl of 1 to 17 carbon atoms, alkenyl of 2 to 17 carbon atoms, unsubstituted or alkyl-substituted cycloalkyl of 5 to 12 carbon atoms, benzyl or phenyl, or $R^4$ and $R^5$ together form $—CH_2—CH_2—$, —CH=CH— or o-phenylene, at from 0 to 110 °C, the batch is heated in the presence of an alcohol of the formula

$$R^6—OH$$

where $R^6$ is alkyl of 1 to 10 carbon atoms, hydroxyethyl or cyclohexyl and $R^3$ and $R^6$ are identical or different, and of a basic compound, the N-(3,5-dichlorophenyl)-oxazolidine-2,4-dione precipitated from the reaction mixture is isolated from the mother liquor at from — 30 to + 90 °C, and additional amounts of N-(3,5-dichlorophenyl)-oxazolidine-2,4-dione are precipitated from the alcoholic mother liquor by adding water, and are isolated from the aqueous liquid at from — 10 to + 60 °C.

2. A process as claimed in claim 1, wherein the reaction is carried out using a compound in which $R^1$ is vinyl or methoxymethyl, $R^2$ is methyl and $R^3$ is alkyl of 1 to 4 carbon atoms.

3. A process as claimed in claim 1, wherein $R^4$ and $R^5$ are each heptyl.

4. A process as claimed in claim 1, wherein the volume ratio of alcoholic mother liquor to water is from 1 : 3 to 3 : 1.

5. A process as claimed in claim 1, wherein, in batchwise production, the N-(3,5-dichlorophenyl)-oxazolidine-2,4-dione which has been separated off from the previous batch by adding water and has been filtered off is mixed, on the filter unit, with the mixture of mother liquor and N-(3,5-dichlorophenyl)-oxazolidine-2,4-dione from the current batch, and the combined product is isolated from the mother liquor of the current batch by filtration.

**Revendications**

1. Procédé de préparation de N-(3,5-dichlorophényl)-oxazolidine-2,4-diones de formule

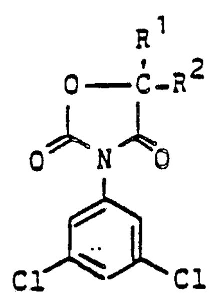

dans laquelle $R^1$ représente un groupe halogénoalcényle à deux atomes de carbone ou alcényle à deux atomes de carbone, chlorométhyle, CN, CO-O-alkyle contenant 2 à 5 atomes de carbone, alcoxyalkyle ou alkylthioalkyle contenant chacun 2 à 4 atomes de carbone et $R^2$ représente un groupe halogénoalcényle à deux atomes de carbone, alcényle à deux atomes de carbone, l'hydrogène ou un groupe méthyle, caractérisé en ce que l'on fait réagir, à des températures de 0 à 110 °C, l'isocyanate de 3,5-dichlorophényle avec des esters glycoliques de formule

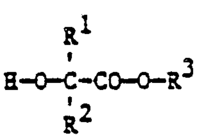

dans laquelle $R^1$ et $R^2$ ont les significations indiquées ci-dessus et $R^3$ représente un groupe alkyle en C 1-C 10 ou un groupe cyclohexyle, en présence de sels stanneux de formule

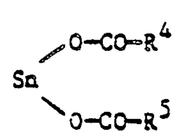

dans laquelle $R^4$ et $R^5$, ayant des significations identiques ou différentes, représentent chacun l'hydrogène, un groupe alkyle en C 1-C 17, un groupe alcényle en C 2-C 17, un groupe cycloalkyle en C 5-C 12 éventuellement alkylé, un groupe benzyle ou phényle, ou bien $R^4$ et $R^5$ forment ensemble un groupe $—CH_2—CH_2—$, —CH=CH— ou o-phénylène, on chauffe en présence d'alcools de formule

$R^6$—OH

dans laquelle $R^6$ représente un groupe alkyle en C 1-C 10, hydroxyéthyle ou cyclohexyle, qui est identique à $R^3$ ou différent de $R^3$, et de composés basiques, on sépare des liqueurs-mères, à des températures allant de — 30 à + 90 °C, les N-(3,5-dichlorophényl)-oxazolidine-2,4-diones qui ont précipité du mélange de réaction et, à partir des liqueurs-mères alcooliques, on précipite par addition d'eau des compléments de N-(3,5-dichlorophényl)-oxazolidine-2,4-diones et on les sépare du liquide aqueux à des températures comprises entre — 10 et + 60 °C.

2. Procédé selon la revendication 1, caractérisé en ce que l'on fait réagir avec des composés dans lesquels $R^1$ représente un groupe vinyle ou méthoxyméthyle, $R^2$ représente un groupe méthyle et $R^3$ représente un groupe alkyle en C 1-C 4.

3. Procédé selon la revendication 1, caractérisé en ce que $R^4$ et $R^5$ représentent des groupes heptyle.

4. Procédé selon la revendication 1, caractérisé en ce que les proportions relatives en volume entre les liqueurs-mères alcooliques et l'eau sont de 1 : 3 à 3 : 1.

5. Procédé selon la revendication 1, caractérisé en ce que, en production discontinue, on mélange sur le dispositif de filtration les N-(3,5-dichlorophényl)-oxazolidine-2,4-diones du dernier chargement, séparées par addition d'eau et filtrées, avec le mélange des liqueurs-mères et des N-(3,5-dichlorophényl)-oxazolidine-2,4-diones du chargement en cours et on les sépare ensemble, par filtration, des liqueurs-mères du chargement en cours.